**Europäisches Patentamt**

**European Patent Office**

**Office européen des brevets**

(19)

(11) Veröffentlichungsnummer: **0 091 403**
**B1**

(12) **EUROPÄISCHE PATENTSCHRIFT**

(45) Veröffentlichungstag der Patentschrift:
**11.03.87**

(51) Int. Cl.⁴: **A 61 B 17/12**

(21) Anmeldenummer: **83810133.5**

(22) Anmeldetag: **31.03.83**

(54) **Staugurt.**

(30) Priorität: **01.04.82 CH 2011/82**

(43) Veröffentlichungstag der Anmeldung:
**12.10.83 Patentblatt 83/41**

(45) Bekanntmachung des Hinweises auf die Patenterteilung:
**11.03.87 Patentblatt 87/11**

(84) Benannte Vertragsstaaten:
**CH DE FR GB IT LI NL SE**

(56) Entgegenhaltungen:
**DE - A - 2 824 037**
**DE - B - 1 275 815**
**US - A - 1 332 442**

(73) Patentinhaber: **Sanimed Vertrieb AG, St. Gallen, Oberer Graben 22, CH-9000 St. Gallen (CH)**

(72) Erfinder: **Kirchner, Georg, Paulinenstrasse 25, D-7145 Markröningen (DE)**

(74) Vertreter: **Wenger, René et al, Hepp & Partner AG Marktgasse 18, CH-9500 Wil (CH)**

## Beschreibung

Die Erfindung bezieht sich auf einen Staugurt mit einem aus einem Gehäuse und einem Klemmkeil bestehenden Klemmschloss und einem Band, welches durch das Klemmschloss durchziehbar und mit seinem einen Ende zur Bildung einer Stauschlaufe in dieses einhängbar ist und ein Klemmschloss für einen Staugurt, wobei ein Band durch das Klemmschloss durchziehbar und mit seinem einen Ende zur Bildung einer Stauschlaufe in das Klemmschloss einhängbar ist.

Staugurte für medizinische Anwendungen, z.B. zum Abbinden des Armes eines Patienten, der genannten Art weisen bei bekannten Ausführungsformen am Klemmschloss einen Schlitz zur selbsthemmenden Durchführung eines elastischen Bandes auf, dessen eines Ende in eine am Klemmschloss angelenkte, federbelastete Klappe zur Bildung der Stauschlaufe einhängbar ist.

Zum Zuziehen der Stauschlaufe, z.B. um einen Arm eines Patienten, wird am freien, aus dem Klemmschloss herausragenden Ende des Bandes gezogen. Zum Lösen der Stauschlaufe wird die Klappe entgegen der Federkraft betätigt, wodurch das eingehängte Bandende ausklinkt.

Bei diesem Staugurt ist nachteilig, dass das Ausklinken des Gurts sofort nach Betätigung der Klappe erfolgt. Dies hat zur Folge, dass während der medizinischen Arbeit die Stärke des Bandzuges nicht dosiert verringert werden kann. Eine solche dosierte Verringerung des Bandzuges, damit also die Stärke des Blutstaues in den Adern, ist oft notwendig. Muss dazu der Staugurt vollständig gelöst und neu zusammengezogen werden, behindert oder verlängert dies die medizinische Arbeit oft in unzulässiger Weise. Insbesondere ist eine Einhandbedienung während fortdauerndem Stauvorgang für die Staugurt-Bedienung mit der genannten Ausführungsform nicht möglich.

Durch die DE-A-2 824 037 ist ein Staugurt bekannt geworden, bei dem eine als zweiarmiger Hebel ausgebildete Klemmlasche den Gurt verspannt, wobei sie durch die Gurtspannung in der Klemmlage gehalten wird. Zum Lösen der Gurtspannung wird die Klemmlasche am freien Hebelende niedergedrückt. Je nach Spannkraft im Gurt, muss zum Lösen jedoch relativ viel Kraft aufgewendet werden, wobei sich der Gurt schlagartig entspannt.

Die DE-B-1 275 815 beschreibt eine Rahmenschnalle für Bänder oder dergleichen, bei der das Band durch ein federbelastetes Klemmstück festgeklemmt wird, das am Rahmen der Schnalle mit einem Achsbolzen befestigt ist und aus der Ebene des Rahmens herausschwenkbar ist. Diese Rahmenschnalle wurde jedoch für Uhrenarmbänder entwickelt und eignet sich nicht für ein rasches Lösen oder Spannen des Bandes.

Aufgabe der Erfindung ist die Schaffung eines Staugurtes bzw. eines Klemmschlosses für einen Staugurt, welcher bzw. welches einhändig bedienbar ist, d.h. dass nach Bildung der Stauschlaufe sowohl das Zusammenziehen derselben wie auch deren dosiertes oder vollständiges Lösen einhändig vorgenommen werden kann.

Diese Aufgabe wird erfindungsgemäss gelöst, indem das Klemmschloss einen gegen die Kraft einer Feder im Gehäuse verschiebbaren Klemmkeil enthält, der in seiner Arbeitslage zufolge der Feder das Band gegen Öffnen der Stauschlaufe flächig klemmend an eine Wand des Gehäuses drückt, und dass durch eine Verschiebung des Klemmkeiles gegen die Kraft der Feder die Klemmung des Bandes lösbar ist bzw. das Klemmschloss einen gegen die Kraft einer Feder im Gehäuse verschiebbaren Klemmkeil enthält, der in seiner Arbeitslage zufolge der Feder das Band gegen eine Wand des Klemmschlosses drückt. Ausserdem kann der Klemmkeil zur Längsachse des Bandes quer liegen oder zur Längsachse des Bandes parallel liegen. Vorteilhafterweise kann zwischen Klemmkeil und dem Band bzw. der Wand des Klemmschlosses eine Klemmplatte im Klemmschloss angeordnet sein. Auch kann das Klemmschloss an der dem Keil gegenüberliegenden Innenseite eine Keilfläche im Bereich des Klemmkeiles aufweisen.

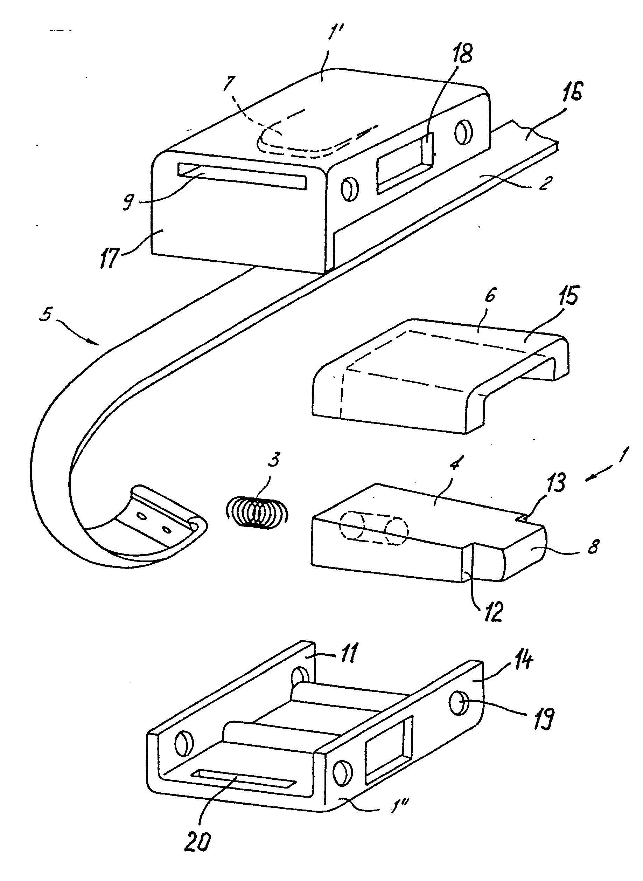

Weitere Einzelheiten und Merkmale ergeben sich aus der Beschreibung eines Ausführungsbeispiels der Erfindung anhand der Zeichnung. In dieser zeigt die einzige Figur die wesentlichen Elemente eines Staugurtes vor dem Zusammenbau (Explosionsdarstellung).

Der Staugurt weist ein Klemmschloss 1 mit einem Ober- und Unterteil 1' bzw. 1'' und ein durch dieses hindurchgezogenes und kraft- und/oder formschlüssig gehaltenes Band 2 auf, welches mit seinem einen Ende zur Bildung einer Stauschlaufe 5 in einem Schlitz des Unterteils 1'' lösbar einhängbar ist. Im Unterteil 1'' ist quer zur Klemmschlosslängsachse ein Klemmkeil 4 quer verschiebbar eingelegt, der mit einem Griffansatz 8 aus dem Unterteil 1'' ragt. Gegenüber dem Griffansatz 8 liegt eine Feder 3 am Klemmkeil 4 an, welche sich gegen den Unterteil 1'' abstützt. Auf dem Klemmkeil 4 liegt eine Klemmplatte 6 höhenverschiebbar zur flächigen Klemmung des zwischen ihr und dem Oberteil 1' hindurchlaufenden Bandes 2 auf. Das Oberteil 1' weist an einer seiner Stirnseiten einen Einlaufschlitz 9 für das Band 2 und an einer seiner schmalen Längsseiten eine Ausnehmung für den Griffansatz 8 des Klemmkeiles 4 auf.

Die in der Zeichnung dargestellten Teile des Klemmschlosses 1 können aufgrund der vorherigen Beschreibung wie folgt zusammengebaut werden:

In den Unterteil 1'' wird der Klemmkeil 4 eingesetzt, indem der Griffansatz 8 desselben von der Innenseite des Unterteils 1'' her gesehen durch ein Fenster 10 des letzteren hindurchgesteckt wird. Gleichzeitig wird die Feder 3 zwischen der dem Griffansatz 8 abgewandten Seite des Klemmkeils 4 und der Seitenwand 11 des Unterteils 1'' eingesetzt, wodurch der Klemmkeil 4 mit Schultern 12 und 13 beidseitig des Griffansatzes 8 gegen die andere Seitenwand 14 des Unterteils 1'' gedrückt wird. Danach wird die Klemmplatte 6 über den Klemmkeil 4 gesetzt, so, dass der Klemmkeil 4 in die Ausnehmung 15 der Klemmplatte 6 zu liegen kommt. Danach wird das Band 2 mit seinem freien Ende 16 durch den Einlaufschlitz 9 des Oberteils 1' und an der gegenüberliegenden Seite wieder hinausgezogen. Der Oberteil 1' mit dem durchgezogenen Band 2 wird dann auf die Klemmplatte 6 aufgesetzt, so, dass der Griffansatz 8 auch durch ein Fenster 18 des Oberteils 1' hindurchragt. Die Öffnungen 19 dienen zum Einsetzen von Befestigungsteilen, damit der Oberteil 1' und der Unterteil 1'' miteinander verbunden werden können.

Bei der Verwendung des Staugurtes wird das Band 2 z.B. um den Oberarm eines Patienten gelegt und mit seinem freien Ende in einen Schlitz 20 im Unterteil 1'' eingehängt. Am freien Bandende wird sodann so lange gezogen, bis die Stauschlaufe eng zusammengezogen ist und eine Stauwirkung beim Arm des Patienten eintritt. Ist der Staueffekt zu gross, insbesondere bei längerdauerndem Stau, dann kann durch dosiertes Eindrücken des Griffansatzes 8 der Klemmkeil 4 etwas verschoben werden. Dadurch wird die Reibung beidseits des Bandes erzeugende Normalkraft verringert und das Band kann etwas in Richtung Stauschlaufe gleiten. Für gänzliches Lösen der Stauschlaufe drückt man den Griffansatz 8 ganz nach innen, löst die Zugspannung im Band und erwirkt so das Aushängen des schlaufenseitigen Bandendes.

In einer anderen Ausführungsform (nicht gezeigt) ist der Klemmkeil in Achsrichtung des Bandes 2 verschiebbar angeordnet. Diese Ausführungsform bietet vor allem den Vorteil, dass ein längerer Klemmkeil verwendet und damit die Stauschlaufe feinfühliger dosiert gelöst werden kann.

In einer weiteren Ausführungsform weist der Oberteil 1' an einer Stelle gegenüber der Klemmplatte 6 eine Keilfläche 7 auf, welche bei entsprechend gewählten Bandmaterialien dieses durch die sich so ergebende Verengung verformt und dadurch teilweise formschlüssig festhält. Anstelle der Keilfläche 7 können Rippen in jeder auch nur annähernd oder diagonal verlaufenden Richtung als Rutschhemmer vorgesehen sein.

**Patentansprüche**

1. Staugurt mit einem aus einem Gehäuse und einem Klemmkeil bestehenden Klemmschloss und einem Band, welches durch das Klemmschloss durchziehbar und mit seinem einen Ende zur Bildung einer Stauschlaufe in dieses einhängbar ist, dadurch gekennzeichnet, dass das Klemmschloss (1) einen gegen die Kraft einer Feder (3) im Gehäuse (1', 1'') verschiebbaren Klemmkeil (4) enthält, der in seiner Arbeitslage zufolge der Feder (3) das Band (2) gegen Öffnen der Stauschlaufe (5) flächig klemmend an eine Wand des Gehäuses (1', 1'') drückt, und dass durch eine Verschiebung des Klemmkeiles (4) gegen die Kraft der Feder (3) die Klemmung des Bandes (2) lösbar ist.

2. Staugurt nach Anspruch 1, dadurch gekennzeichnet, dass der Klemmkeil (4) quer zur Längsachse des Bandes (2) liegt.

3. Staugurt nach Anspruch 1, dadurch gekennzeichnet, dass der Klemmkeil (4) zur Längsachse des Bandes (2) parallel liegt.

4. Staugurt nach Anspruch 2 oder 3, dadurch gekennzeichnet, dass zwischen Klemmkeil (4) und dem Band (2) eine Klemmplatte (6) im Klemmschloss (1) angeordnet ist.

5. Staugurt nach einem der Ansprüche 2, 3 oder 4, dadurch gekennzeichnet, dass das Klemmschloss (1) an der am Band (2) anliegenden Innenseite eine Keilfläche (7) im Bereich des Klemmkeiles (4) aufweist.

6. Klemmschloss für einen Staugurt mit einem Gehäuse und einem Klemmkeil, wobei ein Band durch das Klemmschloss durchziehbar und mit seinem einen Ende zur Bildung einer Stauschlaufe in das Klemmschloss einhängbar ist, dadurch gekennzeichnet, dass das Klemmschloss (1) einen gegen die Kraft einer Feder (3) im Gehäuse verschiebbaren Klemmkeil (4) enthält, der in seiner Arbeitslage zufolge der Feder (3) das Band gegen eine Wand des Klemmschlosses (1) drückt.

7. Klemmschloss nach Anspruch 6, dadurch gekennzeichnet, dass der Klemmkeil (4) quer zur Längsachse des Klemmschlosses (1) liegt.

8. Klemmschloss nach Anspruch 6, dadurch gekennzeichnet, dass der Klemmkeil (4) parallel zur Längsachse des Klemmschlosses (1) liegt.

9. Klemmschloss nach Anspruch 7 oder 8, dadurch gekennzeichnet, dass auf dem Klemmkeil (4) eine Klemmplatte (6) sitzt, welche von diesem gegen eine Wand des Klemmschlosses (1) gedrückt wird.

10. Klemmschloss nach einem der Ansprüche 7, 8 oder 9, dadurch gekennzeichnet, dass die dem Klemmkeil (4) gegenüberliegende Wand des Klemmschlosses (1) eine Keilfläche (7) aufweist.

**Claims**

1. A tourniquet belt including a clamping fastener comprising a housing and a clamping wedge member, and a belt member which can be passed through the clamping fastener and which can be engaged thereinto by means of its one end to form a tourniquet loop, characterised in that the clamping fastener (1) includes a clamping wedge member (4) which is displaceable against the force of a spring (3) in the housing (1', 1'') and which in its operative position, as a result of the spring (3), presses the belt member (2) in clamping engagement over an area against a wall of the housing (1', 1''), to resist opening of the tourniquet loop (5), and that the clamping action in respect of the belt member (2) can be released by displacement of the clamping wedge member (4) against the force of the spring (3).

2. A tourniquet belt according to claim 1 characterised in that the clamping wedge member (4) is disposed transversely with respect to the longitudinal axis of the belt member (2).

3. A tourniquet belt according to claim 1 characterised in that the clamping wedge member (4) is

**0 091 403**

disposed parallel to the longitudinal axis of the belt member (2).

4. A tourniquet belt according to claim 2 or claim 3 characterised in that a clamping plate (6) is disposed in the clamping fastener (1) between the clamping wedge member (4) and the belt member (2).

5. A tourniquet belt according to one of claims 2, 3 and 4 characterised in that at the inward side which bears against the belt member (2) the clamping fastener (1) has a wedge surface (7) in the region of the clamping wedge member (4).

6. A clamping fastener for a tourniquet belt having a housing and a clamping wedge member, wherein a belt member can be pulled through the clamping fastener and can be engaged into the clamping fastener by means of its one end to form a tourniquet loop characterised in that the clamping fastener (1) includes a clamping wedge member (4) which is displaceable against the force of a spring (3) in the housing and which in its operative position as a result of the spring (3) presses the belt member against a wall of the clamping fastener (1).

7. A clamping fastener according to claim 6 characterised in that the clamping wedge member (4) is disposed transversely with respect to the longitudinal axis of the clamping fastener (1).

8. A clamping fastener according to claim 6 characterised in that the clamping wedge member (4) is disposed parallel to the longitudinal axis of the clamping fastener (1).

9. A clamping fastener according to claim 7 or claim 8 characterised in that a clamping plate (6) sits on the clamping wedge member (4) and is pressed by the clamping wedge member (4) against a wall of the clamping fastener (1).

10. A clamping fastener according to one of claims 7, 8 and 9 characterised in that the wall of the clamping fastener (1), which is disposed in opposite relationship to the clamping wedge member (4), has a wedge surface (7).

**Revendications**

1. Tourniquet comportant une fermeture de serrage constituée d'un boîtier et d'un coin de serrage, ainsi qu'une bande pouvant passer dans la fermeture de serrage et être accrochée dans celle-ci par une de ses extrémités pour former un garrot, caractérisé en ce que la fermeture de serrage (1) comporte un coin de serrage (4) coulissant dans le boîtier (1', 1'') à l'encontre de la force d'un ressort (3) et qui, dans sa position de travail, presse la bande (2) contre une paroi du boîtier (1', 1''), sous l'effet du ressort (3) et empêche le garrot (5) de s'ouvrir, et en ce qu'un déplacement du coin de serrage (4) à l'encontre de la face du ressort (3) permet de dégager la bande (2).

2. Tourniquet selon la revendication 1, caractérisé en ce que le coin de serrage (4) s'étend perpendiculairement à l'axe longitudinal de la bande (2).

3. Tourniquet selon la revendication 1, caractérisé en ce que le coin de serrage (4) s'étend parallèlement à l'axe longitudinal de la bande (2).

4. Tourniquet selon la revendication 2 ou 3, caractérisé en ce qu'une plaque de serrage (6) est disposée dans la fermeture de serrage (1) entre le coin de serrage (4) et la bande (2).

5. Tourniquet selon l'une des revendications 2, 3 ou 4, caractérisé en ce que la fermeture de serrage (1) présente sur la face intérieure reposant contre la bande (2) une surface en coin (7) dans la zone du coin de serrage (4).

6. Fermeture de serrage pour un tourniquet comportant un boîtier et un coin de serrage, dans laquelle une bande peut passer à travers la fermeture de serrage et être accrochée dans celle-ci par une de ses extrémités pour former un garrot, caractérisée en ce que la fermeture de serrage (1) comporte un coin de serrage (4) coulissant dans le boîtier à l'encontre de la force d'un ressort (3) et qui, dans sa position de travail, presse la bande contre une paroi de la fermeture de serrage (1) sous l'effet du ressort (3).

7. Fermeture de serrage selon la revendication 6, caractérisée en ce que le coin de serrage (4) s'étend perpendiculairement à l'axe longitudinal de la fermeture de serrage (1).

8. Fermeture de serrage selon la revendication 6, caractérisée en ce que le coin de serrage (4) s'étend parallèlement à l'axe longitudinal de la fermeture de serrage (1).

9. Fermeture de serrage selon la revendication 7 ou 8, caractérisée en ce qu'une plaque de serrage (6) repose sur le coin de serrage (4) et est pressée par celui-ci contre une paroi de la fermeture de serrage (1).

10. Fermeture de serrage selon l'une des revendications 7, 8 ou 9, caractérisée en ce que la paroi de la fermeture de serrage (1) qui est tournée vers le coin de serrage (4) présente une surface en coin (7).